# EUROPEAN PATENT APPLICATION

(11) **EP 4 302 681 A1**
(43) Date of publication of application: **10.01.2024**
(21) Application number: 22763167.8
(22) Date of filing: 28.02.2022
(51) Int. Cl.: A61B 1/045

(54) **MEDICAL IMAGE PROCESSING DEVICE, MEDICAL IMAGE PROCESSING METHOD, AND PROGRAM**

(30) Priority: 04.03.2021 JP 2021034207
(71) Applicant: FUJIFILM Corporation, Tokyo 106-8620 (JP)
(72) Inventor: OOSAKE, Masaaki, Ashigarakami-gun, Kanagawa 258-8538 (JP)
(74) Representative: Hughes, Andrea Michelle
(86) International application number: PCT/JP2022/008168
(87) International publication number: WO 2022/186111

(57) **Abstract**

Provided are a medical image processing apparatus, a medical image processing method, and a program by which a user can efficiently observe a medical image. A processor of the medical image processing apparatus is configured to perform: medical image acquisition processing of sequentially acquiring time-series medical images; first scene recognition processing of recognizing at least one first scene from one medical image of the medical images; second scene recognition processing of recognizing a second scene from the one medical image if the at least one first scene is recognized; first notification processing of providing a notification indicating that the at least one first scene is recognized; and second notification processing of providing a notification indicating that the second scene is recognized.

## Description

### BACKGROUND OF THE INVENTION

### 1. Field of the Invention

The present invention relates to a medical image processing apparatus, a medical image processing method, and a program, and in particular, to techniques of a medical image processing apparatus, a medical image processing method, and a program for assisting a user who observes a medical image.

### 2. Description of the Related Art

A general endoscope apparatus emits illumination light from a distal end of an insertion part of an endoscope, and captures an image of an observation target with a camera to acquire a medical image. The captured medical image is displayed on a monitor, and a user observes the medical image displayed on the monitor and performs an examination.

In recent years, by using a recognizer that is trained through machine learning, it has become possible to recognize a medical image with high accuracy (A. Krizhevsky, I. Sutskever, and G. Hinton. ImageNet classification with deep convolutional neural networks. In NIPS, 2012). Also in an endoscope apparatus, it is considered to automatically recognize a specific scene by using a recognizer that is trained through machine learning and to notify a user of the recognized scene.

For example, JP2020-146202A describes a technique of providing, in accordance with an operation of an endoscope operator, a notification indicating information of interest in a region of interest included in a medical image acquired by an endoscope apparatus.

### SUMMARY OF THE INVENTION

Here, if a user (doctor) wants to observe a specific examination scene, in some cases, it is insufficient to be notified only that the specific examination scene is recognized. If an insertion part of an endoscope apparatus is near the specific examination scene, the user can cause the endoscope apparatus to recognize the specific examination scene relatively immediately. On the other hand, if the insertion part of the endoscope apparatus is away from the specific examination scene, the user has to adjust the imaging position, angle, distance, and the like without any assistance until the endoscope apparatus recognizes the specific examination scene, and in some cases, it takes time until the specific examination scene is recognized.

The present invention has been made in view of such circumstances, and an object thereof is to provide a medical image processing apparatus, a medical image processing method, and a program by which a user can efficiently observe a medical image.

A medical image processing apparatus according to one aspect of the present invention for achieving the above object is a medical image processing apparatus including a processor configured to perform: medical image acquisition processing of sequentially acquiring time-series medical images; first scene recognition processing of recognizing at least one first scene from one medical image of the medical images; second scene recognition processing of recognizing a second scene from the one medical image if the at least one first scene is recognized; first notification processing of providing a notification indicating that the at least one first scene is recognized; and second notification processing of providing a notification indicating that the second scene is recognized.

According to this aspect, the first scene is recognized from the medical image, and the user is notified that the first scene is recognized, the second scene is recognized from the medical image, and the user is notified that the second scene is recognized. Accordingly, since the user is notified that the first scene and the second scene are recognized, the user can grasp where a camera (e.g., an insertion part of an endoscope) that photographs the medical image is located, and can observe the medical image more efficiently.

Preferably, the at least one first scene contains the second scene.

According to this aspect, after being notified that the first scene is recognized, the user can expect the second scene to be recognized, and can observe the medical image more efficiently.

Preferably, the medical image processing apparatus includes a second scene recognizer configured to perform the second scene recognition processing for each of the at least one first scene. The first scene recognition processing recognizes two or more first scenes of the at least one first scene, and in accordance with the two or more first scenes recognized in the first scene recognition processing, the second scene recognizer is selected to recognize the second scene.

Preferably, after the second scene is determined to be recognized in the second scene recognition processing, the first notification processing is not performed.

According to this aspect, once the second scene is recognized and the user is notified, the first notification processing is not performed, which can prevent a number of notifications from being provided and can prevent the observation from being interrupted by repeated notifications.

Preferably, after an image of the second scene is captured, the first notification processing is not performed.

According to this aspect, after the image of the second scene is captured, the first notification processing is not performed, which can prevent a number of notifications from being provided and can prevent the observation from being interrupted by repeated notification.

Preferably, after the second scene is determined to be recognized, the second scene recognition processing is not performed.

According to this aspect, after the second scene is recognized, the recognition processing of the second scene is not performed, and calculation resources can be efficiently used. In addition, this can prevent the observation from being interrupted by repeated notifications as a result of repeated recognition of the same second scene.

Preferably, after an image of the second scene is captured, the second scene recognition processing is not performed.

According to this aspect, after the image of the second scene is captured, the recognition processing of the second scene is not performed, and calculation resources can be efficiently used. In addition, this can prevent the observation from being interrupted by repeated notifications as a result of repeated recognition of the same second scene.

Preferably, the second notification processing continuously provides a notification indicating that the second scene is recognized.

According to this aspect, if there are a plurality of sites to be observed, it is possible to assist the user to comprehensively observe the sites.

Preferably, the first notification processing provides a notification by an indication on a screen, and the second notification processing provides a notification by sound.

Preferably, the indication on the screen is a sample image of the at least one first scene.

Preferably, the first scene recognition processing and the second scene recognition processing are performed by using a Convolutional Neutral Network.

Preferably, the first scene recognition processing recognizes the at least one first scene, based on a classification score.

Preferably, the second scene recognition processing recognizes the second scene, based on a degree of similarity.

Preferably, the at least one first scene and the second scene are scenes in which an image of a site inside a stomach is captured.

A medical image processing method according to another aspect of the present invention is a medical image processing method using a medical image processing apparatus including a processor configured to perform: a medical image acquisition step of sequentially acquiring time-series medical images; a first scene recognition step of recognizing a first scene from one medical image of the medical images; a second scene recognition step of recognizing a second scene from the one medical image if the first scene is recognized; a first notification step of providing a notification indicating that the first scene is recognized; and a second notification step of providing a notification indicating that the second scene is recognized.

A program according to another aspect of the present invention is a program for causing a medical image processing apparatus including a processor to execute a medical image processing method. The program causes the processor to perform: a medical image acquisition step of sequentially acquiring time-series medical images; a first scene recognition step of recognizing a first scene from one medical image of the medical images; a second scene recognition step of recognizing a second scene from the one medical image if the first scene is recognized; a first notification step of providing a notification indicating that the first scene is recognized; and a second notification step of providing a notification indicating that the second scene is recognized.

According to the present invention, the first scene is recognized from the medical image, the user is notified that the first scene is recognized, the second scene is recognized from the medical image, and the user is notified that the second scene is recognized. Accordingly, it is possible to grasp where the camera that captures the medical image is located, and to observe the medical image more efficiently.

### BRIEF DESCRIPTION OF THE DRAWINGS

Fig. 1 is schematic diagram illustrating an overall configuration of an endoscope system;
Fig. 2 is a block diagram illustrating an embodiment of a medical image processing apparatus;
Fig. 3 is a diagram illustrating a specific configuration example of a first scene recognition unit and a second scene recognition unit;
Fig. 4 is a diagram for describing notifications displayed on a display;
Fig. 5 is a diagram for describing notifications displayed on the display;
Fig. 6 is a diagram for describing notifications displayed on the display;
Fig. 7 is a diagram illustrating an example of a display mode of a model image on the display;
Fig. 8 is a flowchart illustrating a medical image processing method;
Fig. 9 is a flowchart illustrating a medical image processing method;
Fig. 10 is a flowchart illustrating a medical image processing method;
Fig. 11 is a display manner in which a first notification unit provides a notification indicating that a first scene is recognized;
Fig. 12 is a display manner in which a second notification unit provides a notification indicating that a second scene is recognized; and
Fig. 13 is a flowchart illustrating a medical image processing method.

### DESCRIPTION OF THE PREFERRED EMBODIMENTS

Hereinafter, preferred embodiments of a medical image processing apparatus, a medical image processing method, and a program according to the present invention will be described with reference to the accompanying drawings.

### Overall Configuration of Endoscope System Including Medical Image Processing Apparatus

### First Embodiment

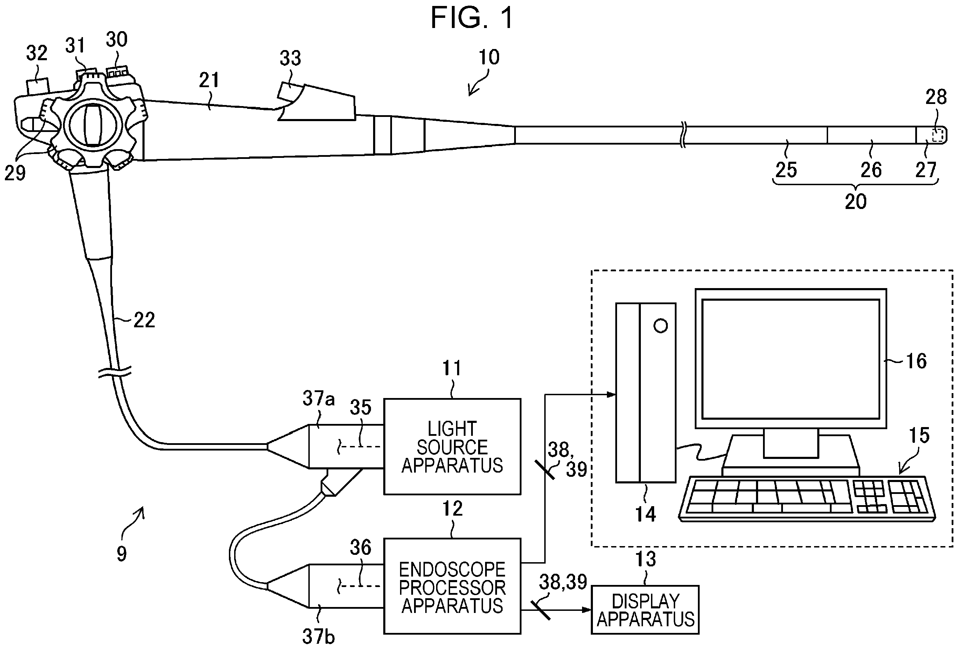

Fig. 1 is a schematic diagram illustrating an overall configuration of an endoscope system including a medical image processing apparatus according to the present invention.

As illustrated in Fig. 1, an endoscope system 9 includes an endoscope 10, which is an electronic endoscope, a light source apparatus 11, an endoscope processor apparatus 12, a display apparatus 13, a medical image processing apparatus 14, an operating unit 15, and a display 16.

The endoscope 10 captures time-series medical images including a subject image and is, for example, a lower or upper digestive tract endoscope. The endoscope 10 has an insertion part 20, a handheld operating unit 21, and a universal cord 22. The insertion part is to be inserted into a subject (e.g., a stomach) and has a distal end and a proximal end. The handheld operating unit 21 is provided continuously from the proximal end side of the insertion part 20 and is held by a doctor, who is a surgeon, to perform various operations. The universal cord 22 is provided continuously from the handheld operating unit 21.

The entirety of the insertion part 20 is formed to have a small diameter and an elongated shape. The insertion part 20 is constituted by continuously providing, in order from the proximal end side to the distal end side thereof, a soft part 25, a bending part 26, and a tip part 27. The soft part 25 has flexibility. The bending part 26 can be bent by an operation of the handheld operating unit 21. In the tip part 27, an imaging optical system (objective lens), an imaging element 28, and the like, which are not illustrated, are incorporated.

The imaging element 28 is an imaging element of a complementary metal oxide semiconductor (CMOS) type or a charge coupled device (CCD) type. Image light of a site to be observed is incident on an imaging surface of the imaging element 28 through an observation window and the objective lens. The observation window, which is not illustrated, is open on a distal end surface of the tip part 27, and the objective lens, which is not illustrated, is disposed behind the observation window. The imaging element 28 captures the image light of the site to be observed, which is incident on the imaging surface (converts the image light into an electric signal) and outputs an image signal. That is, the imaging element 28 sequentially captures medical images. Note that the medical images are acquired as a moving image 38 and a still image 39, which will be described later.

The handheld operating unit 21 is provided with various operating members operated by a doctor (user). Specifically, the handheld operating unit 21 is provided with two types of bending operation knobs 29, an air/water supply button 30, and a suction button 31. The bending operation knobs 29 are used for a bending operation of the bending part 26. The air/water supply button 30 is for air supply/water supply operations. The suction button 31 is for a suction operation. The handheld operating unit 21 is further provided with a still image capturing instruction unit 32 and a treatment tool introduction port 33. The still image capturing instruction unit 32 is for issuing an instruction for capturing the still image 39 of the site to be observed. The treatment tool introduction port 33 is for inserting a treatment tool (not illustrated) into a treatment tool insertion path (not illustrated) penetrating through the insertion part 20.

The universal cord 22 is a connection cord for connecting the endoscope 10 to the light source apparatus 11. The universal cord 22 contains a light guide 35, a signal cable 36, and a fluid tube (not illustrated). The light guide 35, the signal cable 36, and the fluid tube penetrate through the insertion part 20. In addition, an end portion of the universal cord 22 is provided with a connector 37a and a connector 37b. The connector 37a is to be connected to the light source apparatus 11. The connector 37b branches off from the connector 37a and is to be connected to the endoscope processor apparatus 12.

By the connector 37a being connected to the light source apparatus 11, the light guide 35 and the fluid tube (not illustrated) are inserted into the light source apparatus 11. Thus, through the light guide 35 and the fluid tube (not illustrated), necessary illumination light, water, and gas are supplied from the light source apparatus 11 to the endoscope 10. As a result, the site to be observed is irradiated with the illumination light from an illumination window (not illustrated) on the distal end surface of the tip part 27. In accordance with a pressing operation on the above-described air/water supply button 30, the gas or water is injected from an air/water supply nozzle (not illustrated) on the distal end surface of the tip part 27 to the observation window (not illustrated) on the distal end surface.

By the connector 37b being connected to the endoscope processor apparatus 12, the signal cable 36 is electrically connected to the endoscope processor apparatus 12. Thus, through the signal cable 36, an image signal of the site to be observed is output from the imaging element 28 of the endoscope 10 to the endoscope processor apparatus 12, and also, a control signal is output from the endoscope processor apparatus 12 to the endoscope 10.

The light source apparatus 11 supplies the illumination light through the connector 37a to the light guide 35 of the endoscope 10. As the illumination light, light in various wavelength ranges in accordance with an observation purpose, such as white light (light in a white wavelength range or light in a plurality of wavelength ranges), light in one or more specific wavelength ranges, or a combination thereof is selected.

The endoscope processor apparatus 12 controls operations of the endoscope 10 through the connector 37b and the signal cable 36. In addition, based on the image signal acquired from the imaging element 28 of the endoscope 10 through the connector 37b and the signal cable 36, the endoscope processor apparatus 12 generates an image (also referred to as "moving image 38") formed of time-series frame images 38a including the subject image. Furthermore, if the still image capturing instruction unit 32 is operated in the handheld operating unit 21 of the endoscope 10, concurrently with the generation of the moving image 38, the endoscope processor apparatus 12 acquires one frame image 38a in the moving image 38 as the still image 39 in accordance with the timing of an imaging instruction.

The moving image 38 and the still image 39 are medical images obtained by capturing images of the inside of the subject, that is, a living body. In addition, if the moving image 38 and the still image 39 are images obtained with the above-described light in the specific wavelength range (special light), both are special-light images. In addition, the endoscope processor apparatus 12 outputs the generated moving image 38 and the still image 39 to each of the display apparatus 13 and the medical image processing apparatus 14.

Note that the endoscope processor apparatus 12 may generate (acquire) the special-light image having information on the above-described specific wavelength range, based on a usual-light image obtained with the above-described white light. In this case, the endoscope processor apparatus 12 functions as a special-light image acquisition unit. Then, the endoscope processor apparatus 12 obtains a signal in the specific wavelength range by performing calculation based on red, green, and blue (RGB) color information or cyan, magenta, and yellow (CMY) color information included in the usual-light image.

Based on, for example, at least one of the usual-light image obtained with the above-described white light or the special-light image obtained with the above-described light in the specific wavelength range (special light), the endoscope processor apparatus 12 may generate a feature quantity image such as a known oxygen saturation image. In this case, the endoscope processor apparatus 12 functions as a feature quantity image generating unit. Note that each of the moving image 38 and the still image 39 including the above-described in-living-body image, the usual-light image, the special-light image, and the feature quantity image is a medical image obtained by converting results of imaging or measuring of a human body into an image for the purpose of image diagnosis or examination.

The display apparatus 13 is connected to the endoscope processor apparatus 12 and functions as a display unit that displays the moving image 38 and the still image 39 input from the endoscope processor apparatus 12. A doctor (user) operates the insertion part 20 back and forth, for example, while viewing the moving image 38 displayed on the display apparatus 13, and, if a lesion or the like is found at the site to be observed, the doctor (user) operates the still image capturing instruction unit 32 to capture a still image of the site to be observed and give treatment such as diagnosis or biopsy. Note that the moving image 38 and the still image 39 are similarly displayed on the display 16 connected to the medical image processing apparatus 14, which will be described later. In addition, if the moving image 38 and the still image 39 are displayed on the display 16, a notification indication, which will be described later, is also provided together. Accordingly, a user preferably performs diagnosis or the like by viewing what is displayed on the display 16.

### Medical Image Processing Apparatus

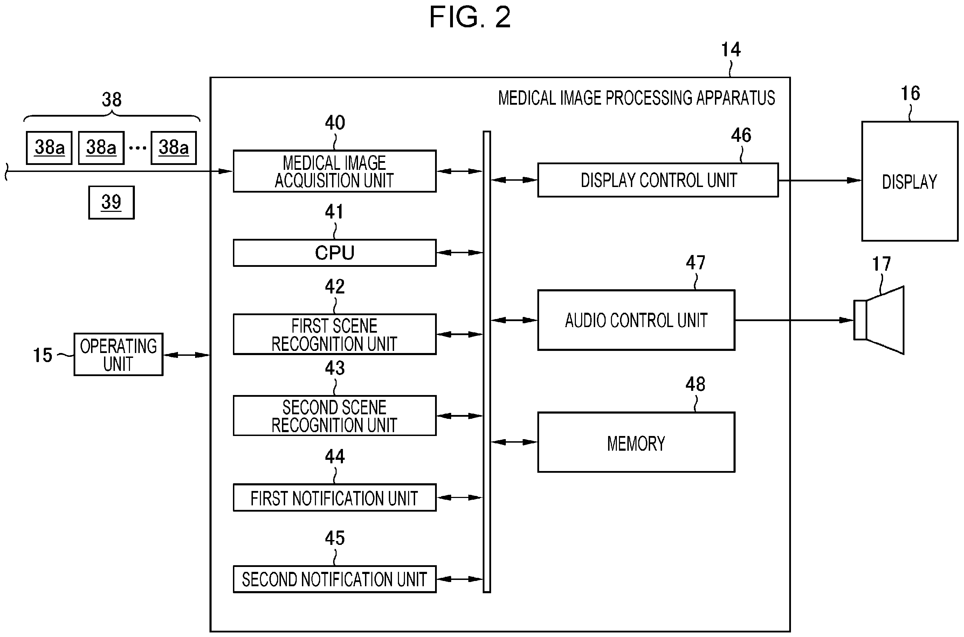

Fig. 2 is a block diagram illustrating an embodiment of the medical image processing apparatus 14. The medical image processing apparatus 14 sequentially acquires time-series medical images and notifies a user that the first scene and the second scene are recognized. The medical image processing apparatus 14 is constituted by, for example, a computer. The operating unit 15 includes, in addition to a keyboard, a mouse, or the like connected to the computer via wired or wireless connection, buttons provided in the handheld operating unit 21 of the endoscope 10, and various monitors, such as a liquid crystal monitor that can be connected to the computer, are used as the display (display unit) 16.

The medical image processing apparatus 14 is constituted by a medical image acquisition unit 40, a central processing unit (CPU) 41, a first scene recognition unit 42, a second scene recognition unit 43, a first notification unit 44, a second notification unit 45, a display control unit 46, an audio control unit 47, and a memory 48. Processing of each unit is implemented by one or more processors. Herein, the processor may be constituted by the CPU 41 or may be constituted by one or more CPUs that are not illustrated.

The CPU 41 operates based on various programs including an operation system and a medical image processing program according to the present invention that are stored in the memory 48, generally controls the medical image acquisition unit 40, the first scene recognition unit 42, the second scene recognition unit 43, the first notification unit 44, the second notification unit 45, the display control unit 46, and the audio control unit 47, and functions as some of these units.

The medical image acquisition unit 40 performs medical image acquisition processing and sequentially acquires time-series medical images. The medical image acquisition unit 40 acquires, from the endoscope processor apparatus 12 (Fig. 1), the time-series medical images including a subject image, by using an image input/output interface, which is not illustrated, connected to the endoscope processor apparatus 12 via wired or wireless connection. In this example, the moving image 38 captured by the endoscope 10 is acquired. In addition, if the above-described still image 39 is captured while the moving image 38 is being captured by the endoscope 10, the medical image acquisition unit 40 acquires the moving image 38 and the still image 39 from the endoscope processor apparatus 12.

The first scene recognition unit 42 performs first scene recognition processing. The first scene herein refers to a scene in a wider range than a second scene described below, and the first scene contains the second scene. For example, if the inside of a stomach is examined with the endoscope apparatus, the first scene is the cardia, the pylorus, the stomach corner, the fundus, the body of the stomach, the pyloric antrum, the lesser curvature, the greater curvature, and the rest. Thus, the first scene can be a scene of each region of an examination target.

The first scene recognition unit 42 recognizes the first scene from an input medical image by various methods. For example, the first scene recognition unit 42 is constituted by a recognizer constituted by a Convolutional Neural Network or the like. The recognizer of the first scene recognition unit 42 learns an image (medical image) in order to recognize the first scene in advance, and recognizes the first scene by using a trained parameter.

The second scene recognition unit 43 performs second scene recognition processing. The medical image recognized as the first scene by the first scene recognition unit 42 is input to the second scene recognition unit 43. The second scene herein refers to a scene that is suitable for observation or diagnosis in the first scene and is a scene in a narrower range than the first scene. For example, the first scene recognition unit 42 recognizes, as the first scene, the cardia inside the stomach, and the second scene recognition unit 43 recognizes, as the second scene, a medical image having a scene that is suitable for observation and in which the cardia is at the center of the image. For example, the first scene recognition unit 42 recognizes, as the first scene, a case where the medical image is blurred due to a movement of the camera or the like or a case where the medical image is dark due to a shielding object, but the second scene recognition unit 43 recognizes, as the second scene, only a case where the medical image is captured at appropriate brightness without blur and shake. The recognizer of the second scene recognition unit 43 learns images (medical images) in advance in order to recognize the second scene, and recognizes the second scene by using a trained parameter.

The first scene recognition unit 42 and the second scene recognition unit 43 may recognize the scenes by determining the input medical image, based on classification or a degree of similarity. If the first scene recognition unit 42 and the second scene recognition unit 43 recognize the scenes by classifying the medical image, the technology described in a literature (B. Zhou, A. Lapedriza, J. Xiao, A. Torralba, and A. Oliva. Learning deep features for scene recognition using places database. In Neural Information Processing Systems (NIPS), pages 487-495, 2014. 1, 4, 6, 8) can be used. In addition, if the first scene recognition unit 42 and the second scene recognition unit 43 recognize the scenes, based on the degree of similarity of a feature quantity of the medical image, the technology described in a literature (FaceNet: A Unified Embedding for Face Recognition and Clustering https://arxiv.org/abs/1503.03832)) can be used.

The first notification unit 44 performs first notification processing and notifies the user that the first scene is recognized. The first notification unit 44 notifies the user that the first scene is recognized, by various methods. For example, the first notification unit 44 provides a notification indicating that the first scene is recognized, on the display 16 via the display control unit 46. Specifically, in a model diagram of an organ drawn in a sub-region of the display 16, the first notification unit 44 displays a region (notification indication) corresponding to the recognized first scene by coloring, flashing, or illuminating the region to notify the user that the first scene is recognized. While the first scene is recognized, the first notification unit 44 may continuously provide the notification indicating that the first scene is recognized, may end providing the notification after providing the notification for a certain period, or may gradually end providing the notification (e.g., the color gradually disappears). Note that although an example in which the first notification unit 44 provides a notification by providing a notification indication on the display 16 has been described above, the notification manner is not limited to this. For example, the first notification unit 44 may provide a notification by using a speaker 17 via the audio control unit 47. In this case, the speaker 17 outputs a notification sound to notify the user that the first scene is recognized.

The second notification unit 45 performs second notification processing and provides a notification indicating that the second scene is recognized. The second notification unit 45 notifies the user that the second scene is recognized, by various methods. For example, the second notification unit 45 provides a notification indicating that the second scene is recognized, on the display 16 via the display control unit 46. Specifically, the second notification unit 45 displays a diagram of the organ drawn in the sub-region of the display 16 by coloring a local region of the diagram. Specifically, in a model diagram of the organ drawn in the sub-region of the display 16, the second notification unit 45 provides a circle (notification indication) in a region corresponding to the recognized second scene and displays the circle in color, or flashes or illuminates the circle, to notify the user that the second scene is recognized. While the first scene is recognized, the first notification unit 44 may continuously provide the notification indicating that the first scene is recognized, may end the notification after providing the notification for a certain period, or may gradually end the notification (e.g., the color gradually disappears). Note that although an example in which the second notification unit 45 provides a notification by providing a notification indication on the display 16 has been described above, the notification manner is not limited to this. For example, the second notification unit 45 may provide a notification by using the speaker 17 via the audio control unit 47. In this case, the speaker 17 outputs a notification sound to notify the user that the second scene is recognized.

The first notification unit 44 and the second notification unit 45 may provide notifications independently of each other, or the first notification unit 44 and the second notification unit 45 may provide notifications in association with each other. If the first notification unit 44 and the second notification unit 45 provide notifications in association with each other, while one of the notifications is being provided, the other of the notifications may be refrained from being provided. In addition, the first notification unit 44 and the second notification unit 45 may provide notifications in different notification manners. For example, the first notification unit 44 may provide a notification by an indication on a screen on the display 16, and the second notification unit 45 may provide a notification by sound output from the speaker 17. In addition, the second notification unit 45 may provide a notification by an indication on the screen on the display 16, and the first notification unit 44 may provide a notification by sound output from the speaker 17.

The display control unit 46 causes the first notification unit 44 or the second notification unit 45 to display the notification indication on the display 16. Specifically, under control of the first notification unit 44, the display control unit 46 causes the display 16 to display a notification indication for providing a notification indicating that the first scene is recognized. In addition, under control of the second notification unit 45, the display control unit 46 causes the display 16 to display a notification indication for providing a notification indicating that the second scene is recognized. In addition, the display control unit 46 generates image data to be displayed, based on the medical images (the moving image 38) acquired by the medical image acquisition unit 40 and outputs the image data to the display 16. Thus, the user is notified that the first scene and the second scene are recognized while observing the medical image.

The audio control unit 47 causes the first notification unit 44 or the second notification unit 45 to reproduce a notification sound from the speaker 17. Specifically, under control of the first notification unit 44, the audio control unit 47 causes the speaker 17 to reproduce a notification sound for providing a notification indicating that the first scene is recognized. In addition, under control of the second notification unit 45, the audio control unit 47 causes the speaker 17 to reproduce a notification sound for providing a notification indicating that the second scene is recognized.

The memory 48 includes a flash memory, a read-only memory (ROM), a random access memory (RAM), a hard disk device, and the like. The flash memory, the ROM, and the hard disk device are non-volatile memories that store an operation system, various programs such as the medical image processing program according to the present invention, the still image 39 that is captured, and the like. In addition, the RAM is a volatile memory from which data can be read and on which data can be written at high speed and that functions as an area for temporarily storing various programs stored in the non-volatile memory and as a work area for the CPU 41.

Next, a specific configuration example of the first scene recognition unit 42 and the second scene recognition unit 43 will be described.

In this example, a case will be described in which seven sites inside the stomach are each observed, and a series of observations are performed in which an image of a representative scene among the respective sites is captured. Specifically, each of the seven sites inside the stomach is recognized as a first scene, and a representative scene, an image of which is to be captured, is recognized as a second scene.

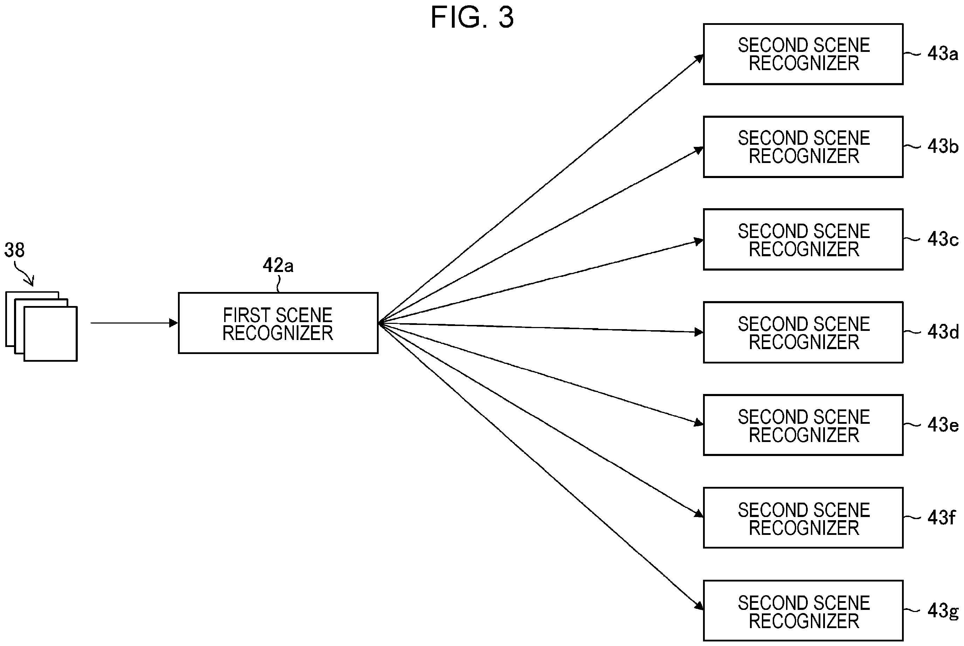

Fig. 3 is a diagram illustrating the specific configuration example of the first scene recognition unit 42 and the second scene recognition unit 43.

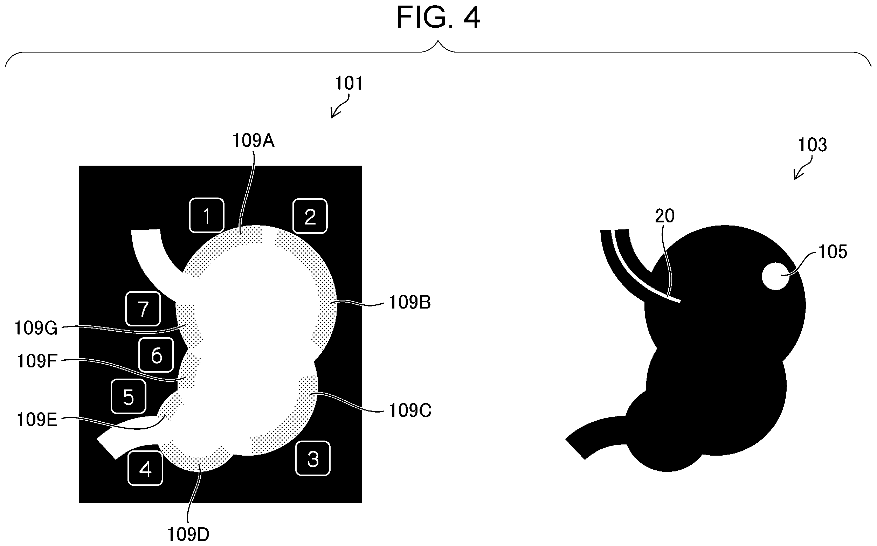

The first scene recognition unit 42 is constituted by a first scene recognizer 42a, and the second scene recognition unit 43 is constituted by second scene recognizers 43a to 43g. The first scene recognizer 42a and the second scene recognizers 43a to 43g are trained models constituted by a convolutional neural network (CNN), which are subjected to machine learning in advance. For example, the first scene recognizer 42a is subjected to learning using learning data constituted by medical images obtained by capturing images of the seven sites inside the stomach so as to recognize respective scenes at the seven sites inside the stomach (see Fig. 4). For example, the second scene recognizers 43a to 43g are subjected to machine learning so as to recognize scenes suitable for image capturing corresponding to the respective seven sites inside the stomach of the first scene. For example, the second scene recognizers 43a to 43g are subjected to learning using learning data constituted by medical images of scenes suitable for capturing images of the seven sites inside the stomach.

The first scene recognizer 42a receives the moving image 38, and recognizes the first scene in each frame image 38a. For example, the first scene recognition unit 42 recognizes the first scene in the frame image 38a, based on a classification score. The first scene recognizer 42a outputs the classification score with respect to the input frame image 38a, and the first scene at a site with the highest classification score is recognized. Upon recognition of the first scene in the frame image 38a, the first scene recognizer 42a transmits the frame image 38a to the second scene recognizers 43a to 43g corresponding to the recognized first scene. For example, upon recognition of the first scene of a second site inside the stomach from the input frame image 38a, the first scene recognizer 42a transmits the frame image 38a to the second scene recognizer 43b corresponding to the second site. Note that as long as no first scene is recognized in the frame image 38a, the first scene recognizer 42a does not transmit the frame image 38a to the second scene recognizers 43a to 43g.

The second scene recognizers 43a to 43g receive the frame image 38a in which the first scene is recognized by the first scene recognizer 42a, and recognize the second scene. For example, the second scene recognizers 43a to 43g recognize the second scene in the frame image 38a, based on the degree of similarity. Specifically, the second scene recognizers 43a to 43g output the degrees of similarity with respect to the input frame image 38a, and recognize the second scene if the output degree of similarity is greater than or equal to a threshold value, and recognizes no second scene if the output degree of similarity is less than the threshold value.

The second scene recognizers 43a to 43g are provided to correspond to the respective seven sites inside the stomach. Specifically, if the first scene recognizer 42a recognizes the first scene of the first site, the frame image 38a recognized as being of the first site is input to the second scene recognizer 43a. In addition, if the first scene recognizer 42a recognizes the first scene of the second site, the frame image 38a recognized as being of the second site is input to the second scene recognizer 43b. In this manner, in accordance with the site recognized by the first scene recognizer 42a, the frame image 38a is input to the corresponding one of the second scene recognizers 43a to 43g.

In the above-described example, the first scene recognizer 42a recognizes a plurality of first scenes, and each of the second scene recognizers 43a to 43g recognizes the second scene in the corresponding one of the first scenes. Thus, with the trained models obtained through machine learning, the first scene recognizer 42a and the second scene recognizers 43a to 43g can be efficiently configured.

Next, specific examples of a first notification indicating that the first scene is recognized and a second notification indicating that the second scene is recognized will be described.

Figs. 4 to 6 are diagrams for describing notifications by indications on the display 16. In Figs. 4 to 6, a model image 101 of a stomach that is an examination target is illustrated, and notification indications corresponding to first to seventh sites of the first scene are illustrated on the model image 101. Specifically, a notification indication 109A corresponding to the first scene of the first site, a notification indication 109B corresponding to the first scene of the second site, a notification indication 109C corresponding to the first scene of the third site, a notification indication 109D corresponding to the first scene of the fourth site, a notification indication 109E corresponding to the first scene of the fifth site, a notification indication 109F corresponding to the first scene of the sixth site, and a notification indication 109G corresponding to the first scene of the seventh site are illustrated on the model image 101. Note that the notification indications 109A to 109G are arranged at positions corresponding to first to seventh sites of the stomach, respectively.

In addition, Figs. 4 to 6 each illustrate a schematic diagram 103 indicating where the insertion part 20 of the endoscope 10 is currently located inside the stomach. Note that the schematic diagram 103 illustrates a target 105 for examination. The target 105 is, for example, a lesion part, a polyp, or the like whose position is identified in advance, and the target 105 is observed or imaged in the examination in this example.

In the case illustrated in Fig. 4, which is a state immediately after the start of an examination of a stomach, as illustrated in the schematic diagram 103, the insertion part 20 is away from the target 105. Thus, in a medical image captured by the imaging element 28 of the insertion part 20, neither first scene nor second scene is recognized, and the notification indications 109A to 109G on the model image 101 are not illuminated. Note that the colors of the notification indications 109A to 109G may be switched for notification, for example, gray for no notification and white or black for notification.

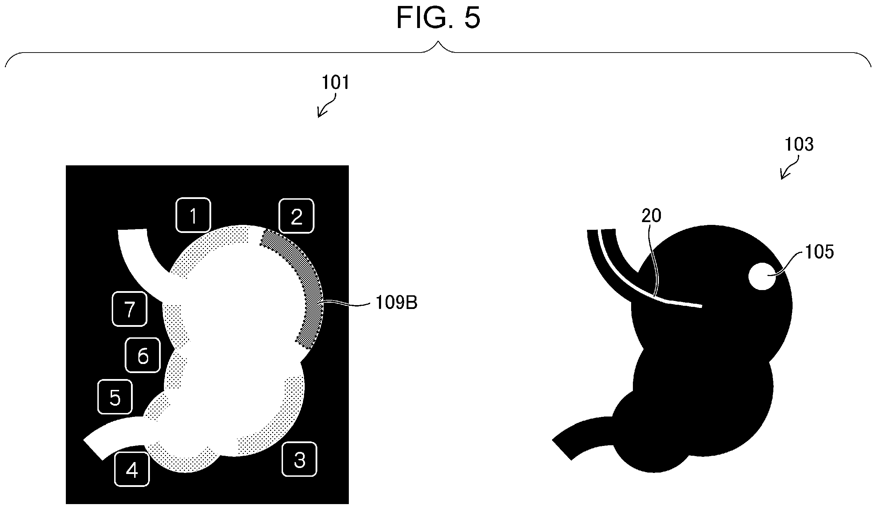

In the case illustrated in Fig. 5, as illustrated in the schematic diagram 103, the insertion part 20 is closer to the target 105. Then, the imaging element 28 captures a medical image having the first scene of the second site including the target 105, and the first scene recognition unit 42 recognizes the first scene of the second site. In addition, since the first scene of the second site is recognized on the model image 101, the notification indication 109B corresponding to the second site is illuminated. Accordingly, the user can understand that the insertion part 20 has moved to the vicinity of the second site where the target 105 is, and the user can be assisted in moving the insertion part 20 to the target 105. Although a notification indicating that the first scene is recognized is provided by illuminating the notification indication 109B in this example, the notification manner is not limited to this. For example, the first notification unit 44 may provide a notification indicating that the first scene is recognized, by causing the display 16 to display a sample image of the first scene.

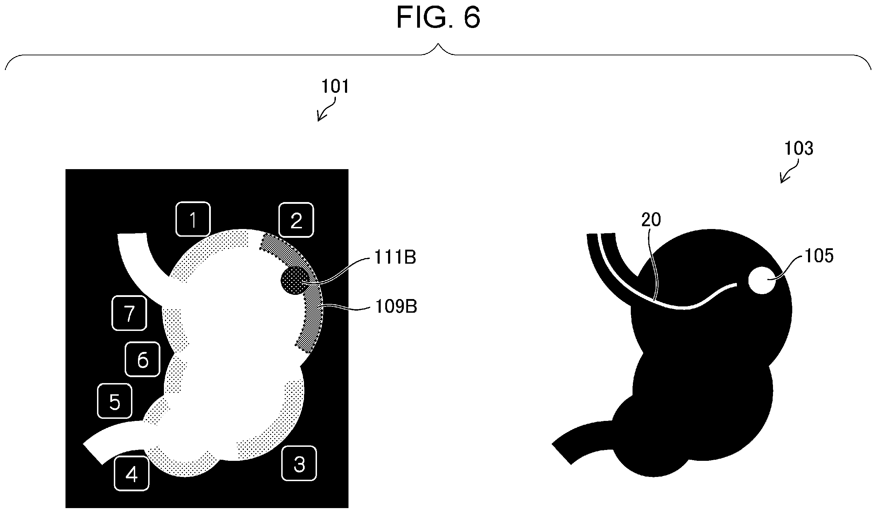

In the case illustrated in Fig. 6, as illustrated in the schematic diagram 103, the insertion part 20 has reached the target 105. Since the insertion part 20 has reached the target 105, the imaging element 28 captures an image of the second scene of the second site, and the second scene recognition unit 43 recognizes the second scene of the second site. Upon recognition of the second scene of the second site on the model image 101, a notification indication 111B of the second scene of the second site is illuminated. Accordingly, the user can grasp that the insertion part 20 has reached the target 105 and the imaging element 28 is in a state of being capable of capturing an image of the second scene of the second site.

Next, an example of a display manner of the above-described model image 101 on the display 16 will be described.

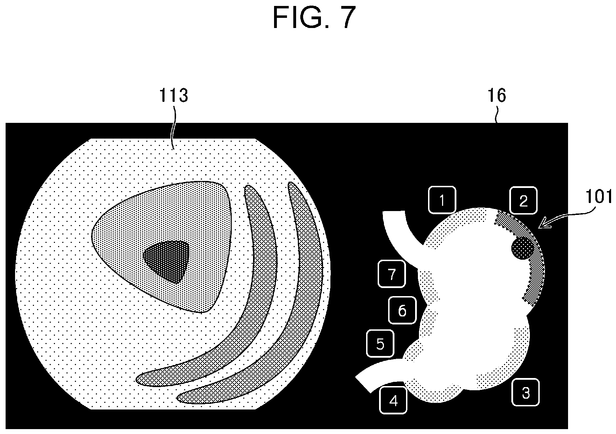

Fig. 7 illustrates an example of the display manner of the model image 101 on the display 16.

As illustrated in Fig. 7, an endoscopic image 113 is displayed in a main region of a display screen of the display 16. The endoscopic image 113 is an image captured by the imaging element 28 of the tip part 27 and is the moving image 38 that is updated as necessary. In addition, the model image 101 is displayed in the sub-region of the display screen of the display 16. Since the model image 101 having the notification indications is displayed in the sub-region of the display 16, the user can grasp the distance between the insertion part 20 and the target 105, and can efficiently perform observation by using the endoscope apparatus.

Next, a medical image processing method performed by using the medical image processing apparatus 14 will be described.

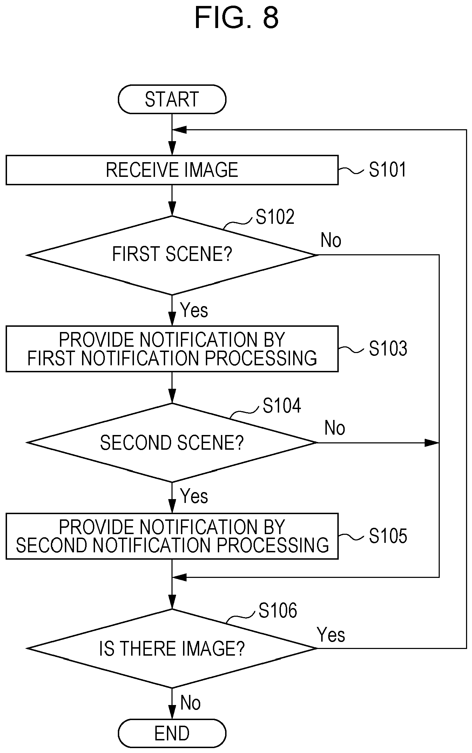

Fig. 8 is a flowchart illustrating the medical image processing method.

The medical image acquisition unit 40 receives a medical image (medical image acquisition step: step S101). Subsequently, the first scene recognition unit 42 recognizes a first scene from the received medical image (first scene recognition step: step S102). If the first scene recognition unit 42 recognizes no first scene, the medical image acquisition unit 40 determines whether there is a subsequent image in time series (step S106). If there is a medical image, the image acquisition unit receives the medical image (step S101). If there is no medical image, the process ends.

On the other hand, if the first scene recognition unit 42 recognizes the first scene, the first notification unit 44 provides a notification indicating that the first scene is recognized (first notification step: step S103). Subsequently, the second scene recognition unit 43 recognizes a second scene from the medical image (second scene recognition step: step S104). If the second scene recognition unit 43 recognizes the second scene, the second notification unit 45 provides a notification indicating that the second scene is recognized (second notification step: step S105). Subsequently, the image acquisition unit determines whether there is a subsequent image (step S106), and if there is a subsequent image, the subsequent medical image is acquired.

As described above, according to this embodiment, the first scene is recognized from the medical image, the user is notified that the first scene is recognized, the second scene is recognized from the medical image, and the user is notified that the second scene is recognized. Accordingly, since the user is notified that the first scene and the second scene are recognized, the user can observe the medical image more efficiently.

### Second Embodiment

Next, a second embodiment will be described. In this embodiment, after the second scene is recognized, the second scene recognition unit 43 does not perform the recognition processing of the second scene in the same first scene. Accordingly, calculation resources can be efficiently used, and it is possible to prevent the observation from being interrupted by repeatedly performing the second notification processing as a result of repeated recognition of the same second scene.

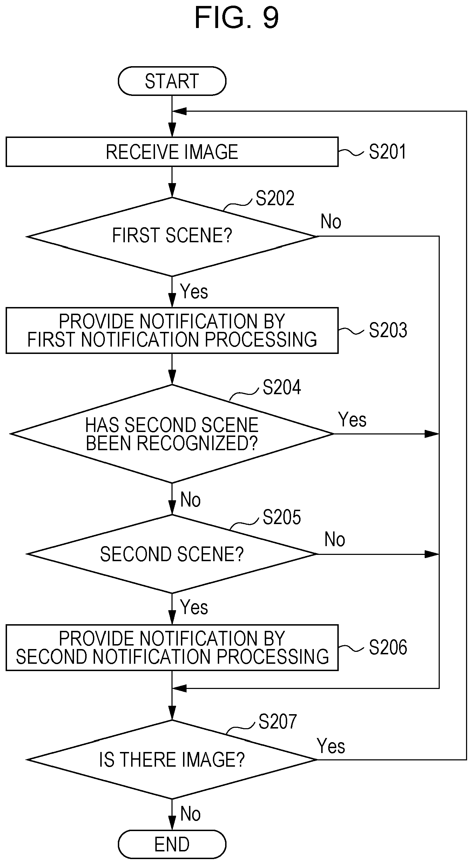

Fig. 9 is a flowchart illustrating a medical image processing method according to this embodiment.

The medical image acquisition unit 40 receives a medical image (step S201). Subsequently, the first scene recognition unit 42 recognizes a first scene from the received medical image (step S202). If the first scene recognition unit 42 recognizes no first scene, the medical image acquisition unit 40 determines whether there is a subsequent image (step S207). If there is a subsequent medical image, the medical image acquisition unit 40 receives the medical image (step S201). If there is no subsequent medical image, the process ends.

On the other hand, if the first scene recognition unit 42 recognizes the first scene, the first notification unit 44 provides a notification indicating that the first scene is recognized (step S203). Subsequently, the second scene recognition unit 43 determines whether a second scene in the recognized first scene has been recognized, based on past recognition records (step S204). Here, if there are a plurality of first scenes (e.g., the examples illustrated in Figs. 3 and 4), the second scene recognition unit 43 (the second scene recognizers 43a to 43g) is provided for each of the first scenes, and thus, the determination is performed for each of the first scenes. If the second scene has been recognized, the second scene recognition unit 43 does not recognize the second scene, and the medical image acquisition unit 40 acquires the subsequent medical image (step S207). Here, it is determined in this example whether the second scene recognition unit 43 has recognized the second scene, based on past recognition records. However, it may be determined whether the second scene recognition unit 43 has recognized the second scene, based on past image capturing records of the second scene. If no second scene has been recognized, the second scene recognition unit 43 recognizes the second scene (step S205). If the second scene recognition unit 43 recognizes the second scene, the second notification unit 45 performs notification processing of indicating that the second scene is recognized (step S206). Subsequently, the medical image acquisition unit 40 determines whether there is a subsequent image (step S207), and if there is a subsequent image, the subsequent medical image is acquired.

As described above, according to this embodiment, if the second scene has been recognized in the past, the second scene recognition unit 43 does not recognize the second scene. Accordingly, calculation resources can be efficiently used, and it is possible to prevent the observation from being interrupted by frequently performing the second notification processing as a result of repeated recognition of the same second scene.

### Third Embodiment

Next, a third embodiment will be described. In this embodiment, the first notification unit 44 and the second notification unit 45 alternatively display a notification indication indicating that the first scene is recognized or a notification indication indicating that the second scene is recognized.

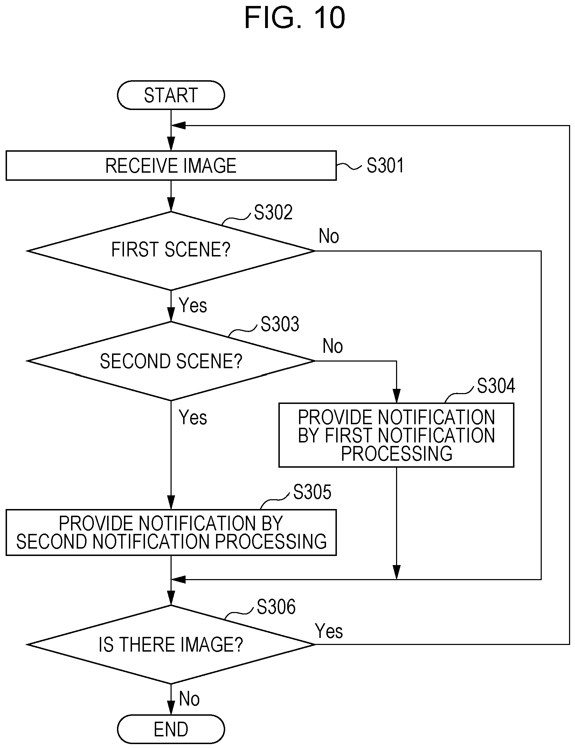

Fig. 10 is a flowchart illustrating a medical image processing method.

The medical image acquisition unit 40 receives a medical image (step S301). Subsequently, the first scene recognition unit 42 recognizes a first scene from the received medical image (step S302). If the first scene recognition unit 42 recognizes no first scene, the medical image acquisition unit 40 determines whether there is a subsequent image in time series (step S306). If there is a medical image, the image acquisition unit receives the medical image (step S301). If there is no medical image, the process ends.

On the other hand, if the first scene recognition unit 42 recognizes the first scene, the second scene recognition unit 43 recognizes a second scene (step S303). If the second scene recognition unit 43 recognizes no second scene, the first notification unit 44 provides a notification indicating that the first scene is recognized (step S304).

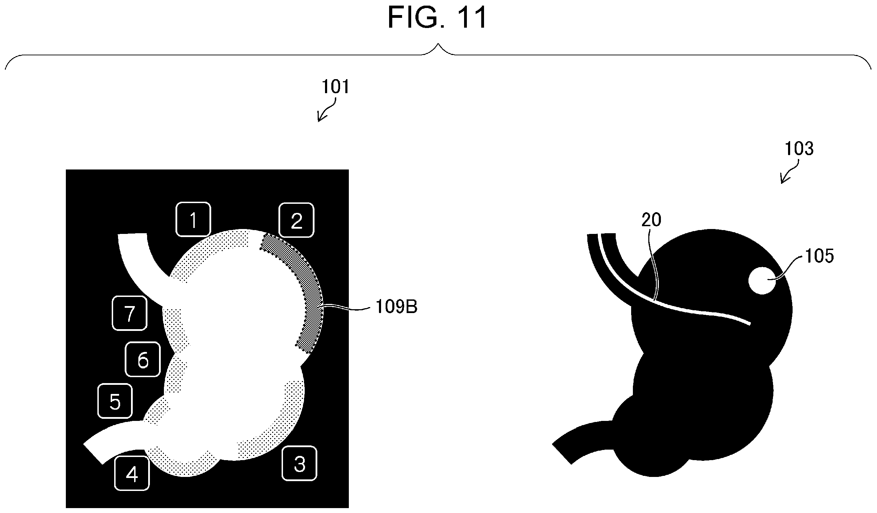

Fig. 11 is a display manner in which the first notification unit 44 provides a notification indicating that the first scene is recognized. Note that portions that have already been described in Fig. 5 are denoted by the same reference numerals, and description thereof is omitted. As illustrated in Fig. 11, the first notification unit 44 notifies the user that the first scene of the second site is recognized, by illuminating the notification indication 109B.

If the second scene recognition unit 43 recognizes the second scene, the second notification unit 45 provides a notification indicating that the second scene is recognized (step S305).

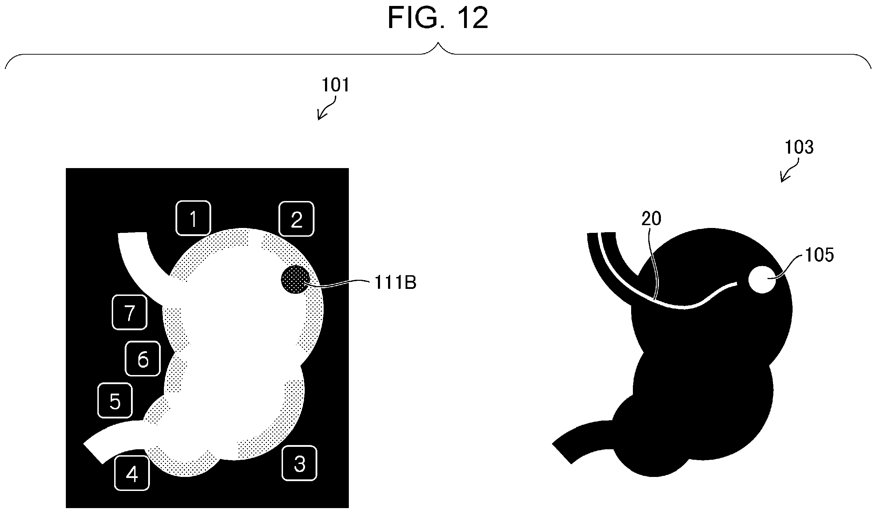

Fig. 12 is a display manner in which the second notification unit 45 provides a notification indicating that the second scene is recognized. Note that portions that have already been described in Fig. 6 are denoted by the same reference numerals, and description thereof is omitted. As illustrated in Fig. 12, the second notification unit 45 notifies the user that the second scene of the second site is recognized, by illuminating the notification indication 111B. Note that in this example, the notification indication 109B indicating that the first scene of the second site is recognized is not illuminated, and only the notification indication 111B indicating that the second scene is recognized is illuminated. In this manner, by alternatively displaying the notification indication indicating that the first scene is recognized or the notification indication indicating that the second scene is recognized, the user can be explicitly notified.

After the first notification unit 44 provides the notification (step S304), or, after the second notification unit 45 provides the notification (step S305), the image acquisition unit determines whether there is a subsequent image (step S306), and if there is a subsequent image, the subsequent medical image is acquired. Note that if the second scene is recognized or an image of the second scene is captured, the first notification unit 44 preferably does not provide a notification even if the corresponding first scene is recognized later. Accordingly, the observation can be prevented from being interrupted by repeated notifications.

As described above, according to this embodiment, the notification indication indicating that the first scene is recognized or the notification indication indicating that the second scene is recognized is alternatively provided, and the user can be explicitly notified. Note that although an example regarding the notification using notification indications has been described in the above example, the notification manner is not limited to this. The first notification unit 44 and the second notification unit 45 may alternatively provide a notification by using audio.

### Fourth Embodiment

Next, a fourth embodiment will be described. In this embodiment, after the single second scene is recognized, the second scene recognition unit 43 does not perform the recognition processing of the second scene. In addition, in this embodiment, the first notification unit 44 and the second notification unit 45 alternatively display a notification indication indicating that the first scene is recognized or a notification indication indicating that the second scene is recognized.

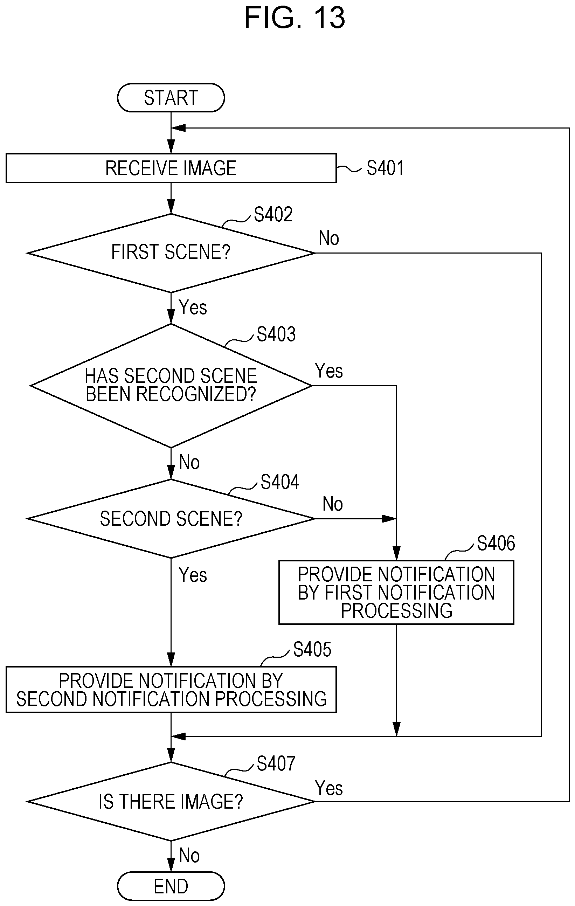

Fig. 13 is a flowchart illustrating a medical image processing method.

The medical image acquisition unit 40 receives a medical image (step S401). Subsequently, the first scene recognition unit 42 recognizes a first scene from the received medical image (step S402). If the first scene recognition unit 42 recognizes no first scene, the medical image acquisition unit 40 determines whether there is a subsequent image (step S407). If there is a subsequent medical image, the medical image acquisition unit 40 receives the medical image (step S401). If there is no subsequent medical image, the process ends.

On the other hand, if the first scene recognition unit 42 recognizes the first scene, the second scene recognition unit 43 determines whether a second scene has been recognized (step S403). If the second scene has been recognized, the second scene recognition unit 43 does not recognize the second scene, and the first notification unit 44 provides a notification indicating that the first scene is recognized (step S406). If no second scene has been recognized, the second scene recognition unit 43 recognizes the second scene (step S404). If the second scene is recognized, the second notification unit 45 provides a notification indicating that the second scene is recognized (step S405). If no second scene is recognized, the first notification unit 44 provides a notification indicating that the first scene is recognized (step S406).

As described above, in this embodiment, after the second scene is recognized, the second scene recognition unit 43 does not perform the recognition processing of the second scene. In addition, in this embodiment, a notification indication indicating that the first scene is recognized or a notification indication indicating that the second scene is recognized is alternatively provided. Accordingly, calculation resources can be efficiently used, and the user can be explicitly notified.

### Miscellaneous

Although the endoscope processor apparatus and the medical image processing apparatus are separately provided in the above embodiments, the endoscope processor apparatus and the medical image processing apparatus may be integrated. That is, the endoscope processor apparatus may be provided with the functions of the medical image processing apparatus.

In addition, the measured examination time or treatment time is stored in the memory within the medical image processing apparatus in association with a diagnosis report or the like, but is not limited to this and may also be stored in an external memory (storage unit) connected to the medical image processing apparatus.

Furthermore, the medical images are not limited to endoscopic images captured by an endoscope and may be, for example, time-series images acquired by another modality such as an ultrasound diagnostic apparatus.

In addition, a hardware configuration that performs various controls of the medical image processing apparatus according to the above embodiments is any of the following various processors. Various processors include a central processing unit (CPU), which is a general-purpose processor that executes software (program) and functions as various control units, a programmable logic device (PLD), which is a processor in which the circuit configuration is changeable after manufacture, such as field programmable gate array (FPGA), a dedicated electric circuit, which is a processor having a circuit configuration that is specially designed to execute specific processing, such as an application specific integrated circuit (ASIC), and the like.

One processing unit may be constituted by one of these various processors, or may be constituted by two or more processors of the same type or different types (e.g., a combination of a plurality of FPGAs or a combination of a CPU and an FPGA). In addition, a plurality of control units may be constituted by one processor. As examples for constituting a plurality of control units by one processor, firstly, there is a form in which one or more CPUs and software are combined to constitute one processor, and this processor functions as a plurality of control units, as typified by a computer such as a client or a server. Secondly, there is a form of using a processor that implements the functions of the entire system including a plurality of control units by using one integrated circuit (IC) chip, as typified by a system on chip (SoC) or the like. In this manner, various control units are constituted by one or more of the above various processors in terms of hardware configuration.

Furthermore, the present invention includes a medical image processing program to be installed in a computer to cause the computer to function as the medical image processing apparatus according to the present invention, and a non-volatile storage medium on which the medical image processing program is recorded.

Although examples in the present invention have been described above, the present invention is not limited to the above-described embodiments, and it is needless to say that various modifications can be made without departing from the gist of the present invention.

### Reference Signs List

- 9: endoscope system
- 10: endoscope
- 11: light source apparatus
- 12: endoscope processor apparatus
- 13: display apparatus
- 14: medical image processing apparatus
- 15: operating unit
- 16: display
- 17: speaker
- 20: insertion part
- 21: handheld operating unit
- 22: universal cord
- 25: soft part
- 26: bending part
- 27: tip part
- 28: imaging element
- 29: bending operation knob
- 30: air/water supply button
- 31: suction button
- 32: still image capturing instruction unit
- 33: treatment tool introduction port
- 35: light guide
- 36: signal cable
- 37a: connector
- 37b: connector
- 38: moving image
- 38a: frame image
- 39: still image
- 40: medical image acquisition unit
- 41: CPU
- 42: first scene recognition unit
- 43: second scene recognition unit
- 44: first notification unit
- 45: second notification unit
- 46: display control unit
- 47: audio control unit
- 48: memory

## Claims

1. A medical image processing apparatus comprising a processor configured to perform:
medical image acquisition processing of sequentially acquiring time-series medical images;
first scene recognition processing of recognizing at least one first scene from one medical image of the medical images;
second scene recognition processing of recognizing a second scene from the one medical image if the at least one first scene is recognized;
first notification processing of providing a notification indicating that the at least one first scene is recognized; and
second notification processing of providing a notification indicating that the second scene is recognized.

2. The medical image processing apparatus according to claim 1, wherein the at least one first scene contains the second scene.

3. The medical image processing apparatus according to claim 1 or 2, comprising a second scene recognizer configured to perform the second scene recognition processing for each of the at least one first scene, wherein
the first scene recognition processing recognizes two or more first scenes of the at least one first scene, and
in accordance with the two or more first scenes recognized in the first scene recognition processing, the second scene recognizer is selected to recognize the second scene.

4. The medical image processing apparatus according to any one of claims 1 to 3, wherein, after the second scene is determined to be recognized in the second scene recognition processing, the first notification processing is not performed.

5. The medical image processing apparatus according to any one of claims 1 to 3, wherein, after an image of the second scene is captured, the first notification processing is not performed.

6. The medical image processing apparatus according to any one of claims 1 to 5, wherein, after the second scene is determined to be recognized, the second scene recognition processing is not performed.

7. The medical image processing apparatus according to any one of claims 1 to 5, wherein, after an image of the second scene is captured, the second scene recognition processing is not performed.

8. The medical image processing apparatus according to any one of claims 1 to 7, wherein the second notification processing continuously provides a notification indicating that the second scene is recognized.

9. The medical image processing apparatus according to any one of claims 1 to 8, wherein
the first notification processing provides a notification by an indication on a screen, and
the second notification processing provides a notification by sound.

10. The medical image processing apparatus according to claim 9, wherein the indication on the screen is a sample image of the at least one first scene.

11. The medical image processing apparatus according to any one of claims 1 to 10, wherein the first scene recognition processing and the second scene recognition processing are performed by using a Convolutional Neutral Network.

12. The medical image processing apparatus according to claim 11, wherein the first scene recognition processing recognizes the at least one first scene, based on a classification score.

13. The medical image processing apparatus according to claim 11 or 12, wherein the second scene recognition processing recognizes the second scene, based on a degree of similarity.

14. The medical image processing apparatus according to any one of claims 1 to 13, wherein the at least one first scene and the second scene are scenes in which an image of a site inside a stomach is captured.

15. A medical image processing method using a medical image processing apparatus comprising a processor configured to perform:
a medical image acquisition step of sequentially acquiring time-series medical images;
a first scene recognition step of recognizing a first scene from one medical image of the medical images;
a second scene recognition step of recognizing a second scene from the one medical image if the first scene is recognized;
a first notification step of providing a notification indicating that the first scene is recognized; and
a second notification step of providing a notification indicating that the second scene is recognized.

16. A program for causing a medical image processing apparatus comprising a processor to execute a medical image processing method, the program causing the processor to perform:
a medical image acquisition step of sequentially acquiring time-series medical images;
a first scene recognition step of recognizing a first scene from one medical image of the medical images;
a second scene recognition step of recognizing a second scene from the one medical image if the first scene is recognized;
a first notification step of providing a notification indicating that the first scene is recognized; and
a second notification step of providing a notification indicating that the second scene is recognized.

17. A non-transitory computer-readable recording medium on which the program according to claim 16 is recorded.
